# EUROPÄISCHE PATENTSCHRIFT

(11) **EP 3 727 215 B1**
(45) Veröffentlichungstag und Bekanntmachung des Hinweises auf die Patenterteilung: **18.08.2021**
(21) Anmeldenummer: 18822308.5
(22) Anmeldetag: 11.12.2018
(51) Int. Cl.: A61F 5/01, A61F 5/37, A61F 5/34

(54) **ORTHOPÄDIETECHNISCHE EINRICHTUNG ZUM STÜTZEN EINER EXTREMITÄT EINES PATIENTEN**
ORTHOPEDIC DEVICE FOR SUPPORTING THE EXTREMITY OF A PATIENT
DISPOSITIF ORTHOPÉDIQUE PERMETTANT DE SUPPORTER L'EXTRÉMITÉ D'UN PATIENT

(30) Priorität: 20.12.2017 DE 102017130685
(43) Veröffentlichungstag der Anmeldung: 28.10.2020
(73) Patentinhaber: Ottobock SE & Co. KGaA, 37115 Duderstadt (DE)
(72) Erfinder: KOPPE, Mario, 37085 Göttingen (DE)
(74) Vertreter: Gramm, Lins & Partner Patent- und Rechtsanwälte PartGmbB
(86) Internationale Anmeldenummer: PCT/EP2018/084359
(87) Internationale Veröffentlichungsnummer: WO 2019/121165

(56) Entgegenhaltungen:
- EP-A1- 1 006 960
- DE-A1-102011 018 470

## Beschreibung

Die Erfindung betrifft eine Einrichtung zum Stützen einer Extremität eines Trägers, wobei die Einrichtung wenigstens ein Stützelement aufweist, auf dem in einem angelegten Zustand der Einrichtung die Extremität aufliegt, wobei das Stützelement eine Stützstruktur und ein Auflageelement aufweist, wobei das Auflageelement ein flächiges, flexibles, vorzugsweise elastisches Material aufweist oder daraus besteht.

Derartige Einrichtungen sind beispielsweise in Form von Orthesen aus dem Stand der Technik seit langem bekannt. Auch bestimmte Formen von Exoskelleten oder anderen Unterstützungseinrichtungen werden im Rahmen der vorliegenden Erfindung unter einer Einrichtung verstanden. Aus der nicht vorveröffentlichten DE 2016 104 880 A1 ist beispielsweise eine Unterstützungsvorrichtung bekannt, mit der das Arbeiten über Kopf erleichtert werden soll, indem eine der Schwerkraft entgegen gerichtete Kraft auf den Oberarm des Trägers ausgeübt wird. Andere Orthesen, beispielsweise Armabduktionsorthesen oder Schulterorthesen sind dazu gedacht, ein Gelenk, beispielsweise einen Ellbogen oder eine Schulter, ruhig zu stellen und eine Bewegung zu verhindern. Insbesondere bei Armobduktionsorthesen muss der Arm in einer angehobenen Position gehalten werden, wozu er auf ein Stützelement aufgelegt wird.

Derartige Stützelemente sind häufig aus einem starren Bauteil hergestellte Schalen oder andere flächige Gegenstände, die gegebenenfalls mit einer Polsterung, beispielsweise einem Kissen oder einem Schaumstoff, belegt sind. Aus der EP 1 060 960 A1 ist eine Stützeinrichtung bekannt, bei der das Stützelement ein luftgefülltes Kissen aufweist. Die DE 10 2011 018470 A1 beschreibt ein System mehrerer verbundener Kissen, die mit Luft gefüllt werden, um einen Druck auf einen darunterliegenden Abschnitt eines Körpers des Trägers der Vorrichtung auszuüben. Dennoch kommt es insbesondere bei Einrichtungen, die über einen langen Zeitraum getragen werden, oftmals zu Druckstellen, unangenehmer Schweißbildung oder sogar der Unterbrechung der Blutzirkulation, da Blutgefäße abgedrückt werden können. Insbesondere aus einem starren Material, beispielsweise Kohlefaserverbundwerkstoff, Kunststoff oder Metall, hergestellte Schalenelemente sind in der Regel nicht an die individuellen Gegebenheiten des jeweiligen Trägers angepasst, sondern weisen eine Standardform auf. Dies ist jedoch nicht die optimale Form, sodass es oftmals zu den genannten Nachteilen kommt.

Der Erfindung liegt daher die Aufgabe zugrunde, eine Einrichtung der eingangs erwähnten Art so weiter zu entwickeln, dass die genannten Nachteile vermieden oder zumindest stark abgeschwächt werden.

Die Erfindung löst die gestellte Aufgabe durch eine Einrichtung gemäß dem Oberbegriff des Anspruchs 1, die sich dadurch auszeichnet, dass das Auflageelement derart an der Stützstruktur befestigt ist, dass sich zwischen dem Auflageelement und der Stützstruktur ein Spalt befindet. Dieser Spalt ist so ausgebildet, dass er auch im angelegten Zustand der Einrichtung vorhanden ist.

Die Extremität des Trägers, beispielsweise ein Bein oder ein Arm, wird auf das Auflageelement des Stützelementes aufgelegt, dass sich die Materialbeschaffenheit in optimaler Weise an die Form des jeweiligen Körperteils anpasst. Auf diese Weise muss auf individuelle Gegebenheiten wenig Rücksicht genommen werden, außer das gegebenenfalls unterschiedlichen Größen der Einrichtung vorgehalten werden müssen. Es ist sichergestellt, dass sich das flächige und flexible Material optimal an die Extremität anformt. Dies wird dadurch erreicht, , dass es nicht vollflächig an der eigentlichen Stützstruktur angeordnet ist, sondern derart, dass sich der Spalt ergibt, in den das Material des Auflageelementes nachgeben und das Auflageelement sich verformen kann, wenn es durch ein Körperteil belastet wird. Der Spalt ist dabei so dimensioniert, dass er durch die Verformung des Auflageelementes zwar kleiner werden kann, jedoch durch die im bestimmungsgemäßen Gebrauch auftretenden Kräfte nicht geschlossen wird. Diese Kräfte werden durch die durch das Stützelement gestützte Extrem ität aufgebracht.

Das Auflageelement ist vorzugsweise an mehreren voneinander beabstandeten Befestigungsstellen mit der Stützstruktur verbunden. Dabei ist es von Vorteil, wenn die Befestigungsstellen auf zwei einander gegenüberliegenden Seiten der Extremität angeordnet sind. Soll durch die Einrichtung beispielsweise ein Oberarm gestützt werden, ist es von Vorteil, die Befestigungsstellen auf zwei gegenüberliegenden Seiten des Armes, beispielsweise auf der medialen und der lateralen Seite, anzuordnen, sodass sich dazwischen das flächige flexible Material des Auflageelementes erstreckt. Die Extremität des Trägers, die auf das Auflageelement aufgelegt wird, kommt somit vorteilhafterweise nicht in Kontakt mit den Befestigungsstellen und insbesondere auch zwischen den Befestigungsstellen nicht in Kontakt mit der eigentlichen Stützstruktur. Druckstellen werden auf diese Weise ebenso sicher vermieden wie die Unterbrechung der Blutzirkulation. Hinzu kommt, dass das flächige flexible Material, dass vorteilhafterweise elastisch ausgebildet ist, wirklich leichter ist als beispielsweise eine aus Metall oder einem Kunststoff hergestellte Schale.

Vorzugsweise ist das Auflageelement an wenigstens drei, bevorzugt wenigstens vier, besonders bevorzugt wenigstens sechs Befestigungsstellen an der Stützstruktur befestigt oder an einer umlaufenden Befestigungsstelle, bevorzugt einem umlaufenden Rand der Stützstruktur, befestigt. Dabei sind auf wenigstens einer, vorteilhafterweise auf beiden Seiten der Extremität, also beispielsweise medial und lateral, jeweils wenigstens zwei Befestigungsstellen angeordnet. Auf diese Weise wird eine optimale Lagerung der Extremität auf dem Auflageelement sichergestellt.

Alternativ kann das Auflageelement auch an einer einzigen Befestigungsstelle mit der Stützstruktur verbunden, wenn sich diese Befestigungsstelle über einen Teil des Umfanges des Auflageelementes, vorzugsweise über den gesamten Umfang des Auflageelementes erstreckt.

In einer bevorzugten Ausgestaltung ist das Auflageelement zumindest teilweise, vorzugsweise jedoch vollständig, aus einem luftdurchlässigen Material hergestellt. Auf diese Weise wird eine unangenehme Schweißbildung verhindert und eine ausreichende Belüftung gewährleistet. Dazu ist es insbesondere von Vorteil, wenn ein Abstand zwischen dem Auflageelement und der Stützstruktur so gewählt wird, sodass auch hier eine Luftzirkulation stattfinden kann. Dies gilt selbstverständlich nicht an den Befestigungsstellen, da an diesen Stellen das Auflageelement an der Stützstruktur befestigt ist.

Vorteilhafterweise ist das Auflageelement zumindest teilweise, vorteilhafterweise jedoch vollständig aus einer Folie, einem Netz, einem Textil beispielsweise einem luftdurchlässigen Gewirk, einem perforierten Polstermaterials, beispielsweise einem Schaumstoff oder einem Elastomer, oder einem auxetischen Material hergestellt. Insbesondere ein auxetisches Material muss nicht elastisch ausgebildet sein, sondern kann auch nur flexibel sein. Es kann beispielsweise aus TPE hergestellt sein. Bei der Folie hat es sich als vorteilhaft herausgestellt, wenn diese perforiert, also insbesondere mit einem Lochmuster versehen ist, um beispielsweise eine Luftzirkulation zu gewährleisten und aus der Folie ein luftdurchlässiges Material zu machen. Dies ist bei einem Netz oder einem Textil nicht notwendig, da diese Strukturen bereits von sich aus über ausreichend Durchbrüche verfügen, die eine Luftzirkulation ermöglichen.

Vorzugsweise ist die Stützstruktur ein Kunststoffbauteil, ein Metallbauteil oder ein Bauteil aus einem Faserverbundwerkstoff oder weist ein solches auf. Die Befestigungsstellen stehen vorzugsweise vom Rest der Stützstruktur in Form von Laschen hervor. Vorzugsweise ist das Auflageelement lösbar an der Stützstruktur befestigt. Dies kann beispielsweise über Klettverschlüsse, Druckknopfelemente, sonstige Formschlusselemente oder durch Klebeverbindungen geschehen. Durch die lösbare Verbindung kann bei Nichtbenutzen der Einrichtung das eigentliche Auflageelement von der Stützstruktur entfernt und beispielsweise gewaschen werden, um eine erneute Verwendung der Einrichtung zu ermöglichen. Selbstverständlich kann auch ein Auflageelement auf diese Weise durch ein anderes Auflageelement ersetzt werden, dass beispielsweise aus einem anderen Material besteht, eine andere Festigkeit aufweist oder andere physikalische und/oder chemische Eigenschaften aufweist. So ist es beispielsweise möglich, ein Gewebe vorzusehen, dessen Fasern zumindest teilweise aus Silber bestehen oder mit Silber beschichtet sind, um eine antibakterielle Wirkung zu erreichen.

Vorzugsweise ist der Abstand zwischen verschiedenen Befestigungsstellen einstellbar, wozu vorteilhafterweise ein Seilsystem oder Zugsystem verwendet wird. Durch die Einstellung des Abstandes zwischen den einzelnen Befestigungsstellen wird die Spannung des flächigen und flexiblen, vorzugsweise elastischen Materials des Auflageelementes, das sich zwischen den Befestigungsstellen erstreckt, verändert. Auf diese Weise kann die Spannung und die Spannungsverteilung individuell auf die optimale Verteilung angepasst werden.

Vorzugsweise verfügt die Einrichtung über wenigstens einen Aktuator, der vorteilhafterweise angetrieben ist, wobei durch den Aktuator ein Abstand zwischen den wenigstens zwei Befestigungsstellen veränderbar ist. Der wenigstens eine Aktuator kann dabei beispielsweise hydraulisch, pneumatisch oder auf andere Weise angetrieben werden. Auf diese Weise ist es möglich, beispielsweise unterschiedliche Spannungen und Spannungsverteilungen in dem flächigen flexiblen Material des Auflageelementes in zeitlicher Abfolge einzustellen und so beispielsweise die Extremität, die auf dem Auflageelement aufliegt, um zu Lagern und die Druckverteilung zu verändern. Dies ist insbesondere für Einrichtungen von Vorteil, die über einen sehr langen Zeitraum getragen werden und die Extremität des Trägers stützen. Auf diese Weise wird beispielsweise die Ausbildung eines Dekubitus verhindert. Die Einstellbarkeit kann auch über wenigstens ein Zugelement erreicht werden, wobei die durch das Zugelement übertragene Zugkraft beispielsweise durch ein drehbares Betätigungselement einstellbar ist. Ein derartiges Zugelement ist beispielsweise aus der EP 2 076 224 B1 bekannt.

In einer bevorzugten Ausgestaltung verfügt die Einrichtung über wenigstens ein zweites Stützelement, das im angelegten Zustand der Einrichtung auf einer dem ersten Stützelement abgewandten Seite der Extremität angeordnet ist. Ein derartiges zweites Stützelement kann identisch oder unterschiedlich zum ersten Stützelement ausgebildet sein um jegliche Ausgestaltung annehmen, die auch für das erste Stützelement beschrieben wurde. Durch die Anordnung auf zwei einander gegenüberliegenden Seiten der Extremität ist eine umfassende Lagerung möglich. So ist beispielsweise bei einem herunterhängenden Arm ein erstes Stützelement auf der hinteren Seite (ventral) angeordnet, dessen Befestigungsstellen sich rechts und links (medial und lateral) vom Oberarm befinden. Ein zweites Stützelement ist frontal zum Oberarm angeordnet, dessen Befestigungsstellen sich ebenfalls medial und lateral, also rechts und links, des Oberarms befinden. Auf diese Weise ist eine nahezu voll umfängliche Lagerung der Extremität im vorliegenden Beispiel also des Oberarms, möglich. Dies ist insbesondere dann von Vorteil, wenn die Extremität in unterschiedlichen Positionen relativ zur Schwerkraftrichtung gelagert werden soll, da auch der Träger unterschiedliche Positionen einnehmen kann.

Vorzugsweise handelt es sich bei der Einrichtung um eine orthopädietechnische Einrichtung, beispielsweise eine Orthese, eine Lagerungsvorrichtung, ein Rehabilitationsgerät oder einen Teil eines Exoskelettes.

Vorzugsweise ist an dem Auflageelement wenigstens ein Sensor und/oder wenigstens eine Elektrode angeordnet. Der wenigstens eine Sensor und/oder die wenigstens eine Elektrode kommt im angelegten Zustand der Einrichtung mit der Extremität und insbesondere der Haut des Trägers in Kontakt. Auf diese Weise können über eine Elektrode elektrische Signale, beispielsweise myoelektrische Signale erfasst und an eine elektrische Steuerung oder eine Auswerteeinheit übermittelt werden. Alternativ oder zusätzlich können auch elektrische Signale an die Haut des Trägers abgegeben werden, um beispielsweise unter der Haut liegende Muskeln zu stimulieren. Wir ein Sensor verwendet kann beispielsweise der Druck bestimmt und so ermittelt werden, ob eine ausreichende Polsterung vorhanden ist. Alternativ oder zusätzlich kann auch eine Feuchtigkeit der Haut, eine Position im Raum, ein Absolutwinkel oder eine andere Größe bestimmt werden, die dann zur Überwachung der Einrichtung und/oder Steuerung von Funktionen der Einrichtung verwendet werden kann.

Um eine Druckstelle zu vermeiden kann das Auflageelement an einer gewünschten Stelle abgespannt sein, um für eine punktuelle Entlastung zu sorgen. Dies ist beispielswiese sinnvoll, wenn die Extremität des Trägers der Einrichtung über knöcherne Vorsprünge oder empfindliche Stellen, beispielsweise eine Wunde, eine Ulzeration oder andere entlastungsbedürftige Areale, verfügt. Zur Abspannung können Stifte, Züge oder flexible und/oder elastische Einheiten verwendet werden.

Mit Hilfe der beiliegenden Zeichnungen werden nachfolgend Ausführungsbeispiele der vorliegenden Erfindung näher erläutert. Es zeigt
- Figur 1: - die schematische Seitenansicht eines Teils einer Einrichtung gemäß einem ersten Ausführungsbeispiel der vorliegenden Erfindung mit zwei Schnittdarstellungen,
- Figur 2: - eine Einrichtung in Form einer Armorthese,
- Figur 3: - die schematische Darstellung einer angelegten Einrichtung gemäß einem weiteren Ausführungsbeispiel der vorliegenden Erfindung,
- Figuren 4 und 5: - die schematischen Darstellungen einer weiteren Ausführungsform im geöffneten und im geschlossenen Zustand,
- Figuren 6 und 7: - schematische Seiten- und Schnittdarstellungen eines Teils einer Einrichtung gemäß einem weiteren Ausführungsbeispiel der vorliegenden Erfindung,
- Figuren 8 und 9: - schematische Darstellungen einer Einrichtung gemäß einem weiteren Ausführungsbeispiel der vorliegenden Erfindung.

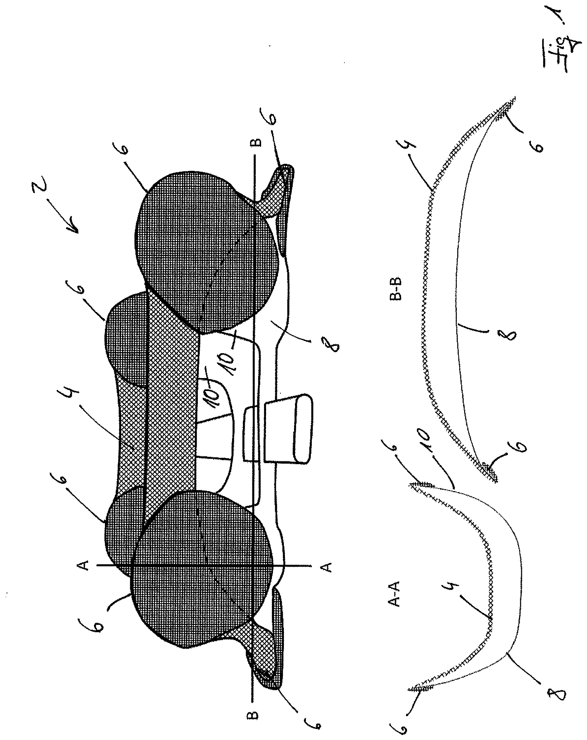

Figur 1 zeigt im oberen Bereich eine schematische 3D-Ansicht eines Stützelementes 2 für eine Einrichtung gemäß einem ersten Ausführungsbeispiel der vorliegenden Erfindung. Das Stützelement verfügt über ein Auflageelement 4, das an sechs Befestigungsstellen 6 an einer Stützstruktur 8 befestigt ist. Die einzelnen Befestigungsstellen 6 sind als separater Bauteile am Rest der Stützstruktur 8 angeordnet. Im unteren Bereich der Figur 1 sind die beiden Schnittdarstellungen entlang der Linie A-A und B-B dargestellt.

Im linken unteren Bereich der Figur 1 ist die Schnittdarstellung entlang der Linie A-A dargestellt. Man erkennt die in diesem Schnitt bogenförmige Stützstruktur 8, an deren oberen Enden sich die Befestigungsstellen 6 befinden. An diesen ist das Auflageelement 4 angeordnet. Im Bereich rechts unten den Figur 1 ist die Schnittdarstellung entlang der Linie B-B dargestellt. Auch hier erkennt man die Stützstruktur 8, die in dieser Schnittdarstellung jedoch umgekehrt gekrümmt ist. Auch hier befinden sich am Ende zwei Befestigungsstellen 6, an denen das Auflageelement 4 angeordnet ist. Wird nun in das Stützelement 2 gemäß Figur 1 eine Extremität, beispielsweise ein Arm eines Trägers eingelegt, kann sich das Auflageelement 4 optimal an die geometrischen Formen und Konturen der Extremität anpassen. Vorzugsweise sind die Befestigungsstellen 6 an Vorsprüngen 10 der Stützstruktur 8 angeordnet, sodass eine Elastizität zumindest im geringen Maße, vorteilhafterweise jedoch bereits durch das Material der Stützstruktur 8 selbst, erreicht wird.

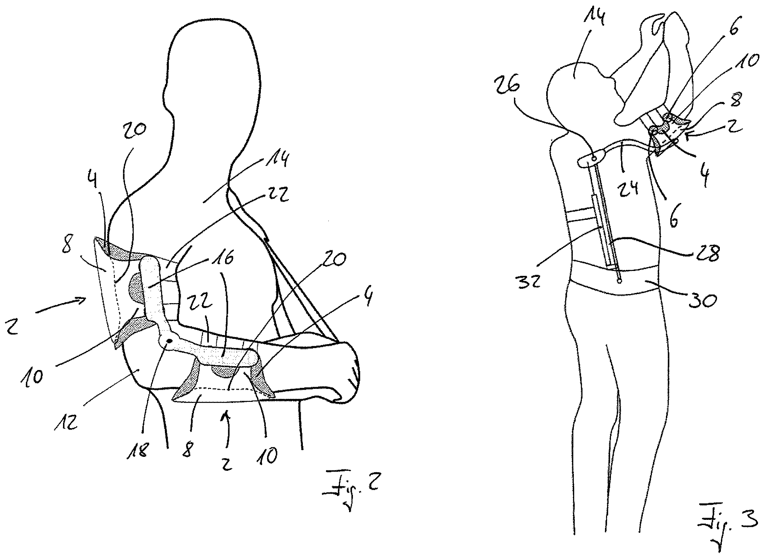

Figur 2 zeigt die Darstellung einer Einrichtung, die an einem Arm 12 eines Trägers 14 angeordnet sind. Die Einrichtung verfügt über zwei Stützelemente 2, von denen eines am Oberarm und eines am Unterarm des Trägers 14 angeordnet sind. Beide sind über Schienen 16 und ein dazwischenliegendes Gelenk 18 miteinander verbunden.

Man erkennt die beiden Auflageelemente 4, die wieder in Form von Netzstrukturen ausgebildet sind und sich an den Arm 12 des Trägers 14 anlegen. Die Stützstruktur 8 verfügt jeweils über Vorsprünge 10, an denen sich die Befestigungsstellen 6 befinden.

Jeweils durch eine gestrichelte Linie 20 ist die Außenkontur des Oberarms und Unterarms des Trägers 14 dargestellt. Man erkennt, dass sie einen Abstand zur Stützstruktur 8 aufweist, sodass der Arm 12 des Trägers 14 nicht mit gegebenenfalls starren oder steif ausgebildeten Bauteilen der Stützelemente 2 in Kontakt kommt. Der einzige Kontakt besteht vorteilhafterweise auf dieser Seite des jeweiligen Arms 12 ausschließlich zum Auflageelement 4. Über Riemen 22 wird die Einrichtung am Träger 14 gehalten.

Figur 3 zeigt eine weitere Ausführungsform einer Einrichtung, die über ein Stützelement 2 verfügt, dass weder die Stützstruktur 8 mit den Vorsprüngen 10 sowie die Befestigungsstellen 6 aufweist, und denen das eigentliche Auflageelement 4 wieder in Form einer netzartigen Struktur, angeordnet ist. Die Einrichtung gemäß Figur 3 verfügt über ein Hebelelement 24, das über ein Gelenk 26 mit einer Kraftübertragungsvorrichtung 28 verbunden ist, die eine aufzubringende Kraft in ein Gürtelelement 30 und damit den Träger 14 überträgt. Zwischen dem Hebelelement 24 und dem Kraftübertragungselement 28 befindet sich ein Kraftaufbringelement 32, durch das eine Kraft auf das Hebelelement 24 und damit auf das Stützelement 2 aufgebracht werden kann.

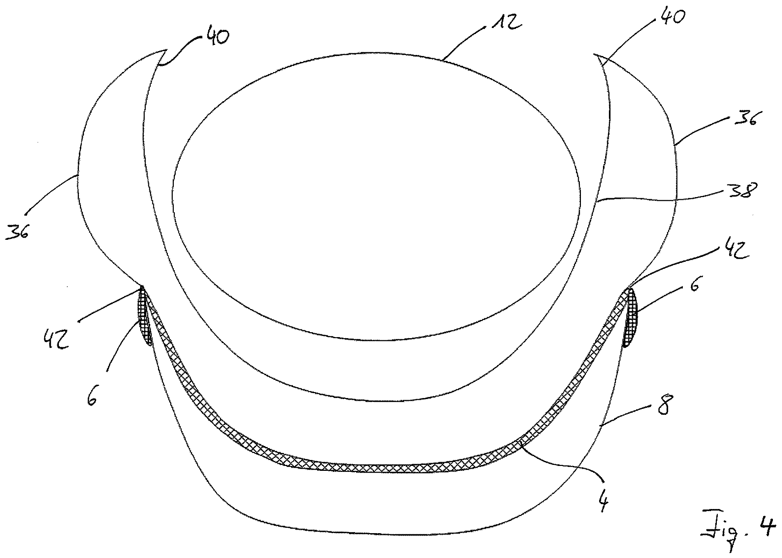

Figur 4 zeigt die Stützstruktur 8 mit dem daran befestigten Auflageelement 4 in einer weiteren Ausgestaltung gemäß der vorliegenden Erfindung. Das Auflageelement 4 ist über die Befestigungsstellen 6 an der Stützstruktur 8 angeordnet. Zusätzlich verfügt die Einrichtung über zwei Schalenelemente 36, zwischen denen sich ein Betätigungselement 38 befindet. Zudem ist ein Arm 12 des Trägers schematisch dargestellt. Dieser Arm 12 kann von oben durch die Öffnung zwischen den beiden Schalenelementen 36 eingeführt werden. Dabei wird er in Figur 4 nach unten bewegt und kommt so mit dem Betätigungselement 38 in Kontakt, dessen Endbereiche 40 an dem Schalenelement 36 befestigt sind. Wird der Arm 12 aus der in Figur 4 gezeigten Situation weiter nach unten bewegt, wird auf das Betätigungselement 38 eine nach unten wirkende Kraft ausgeübt, die dafür sorgt, dass die beiden Schalenelemente 36 um Gelenke 42 herum verschwenkt werden.

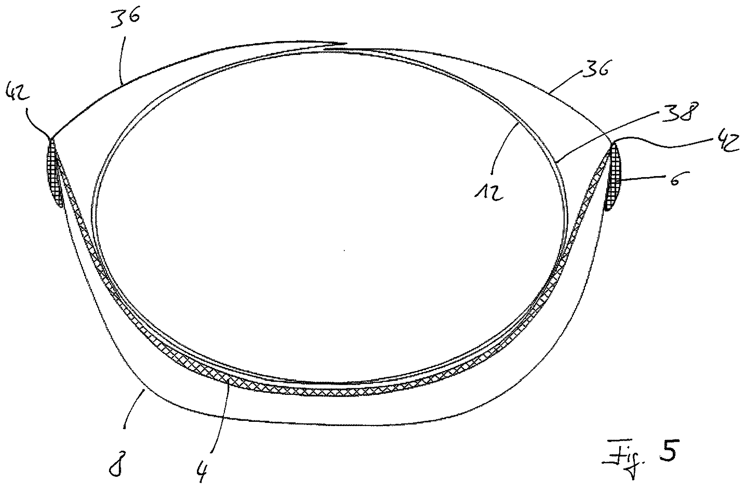

Auf diese Weise wird die in Figur 5 gezeigte Situation erreicht. Der Arm 12 ist nahezu vollständig vom Betätigungselement 38 umgeben. Durch seine Bewegung nach unten hat er die beiden Schalenelemente 36 um die Gelenke 42 herum verschwenkt und so die Anordnung geschlossen.

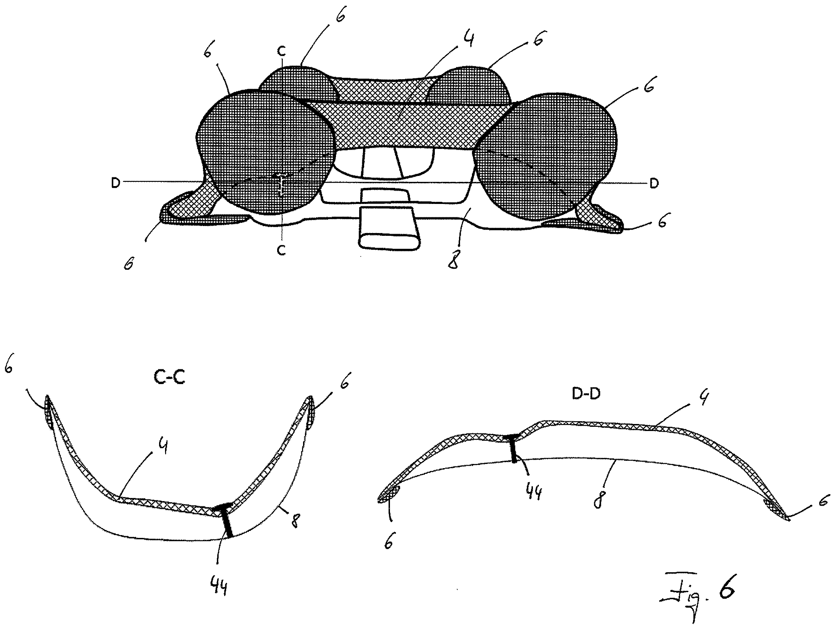

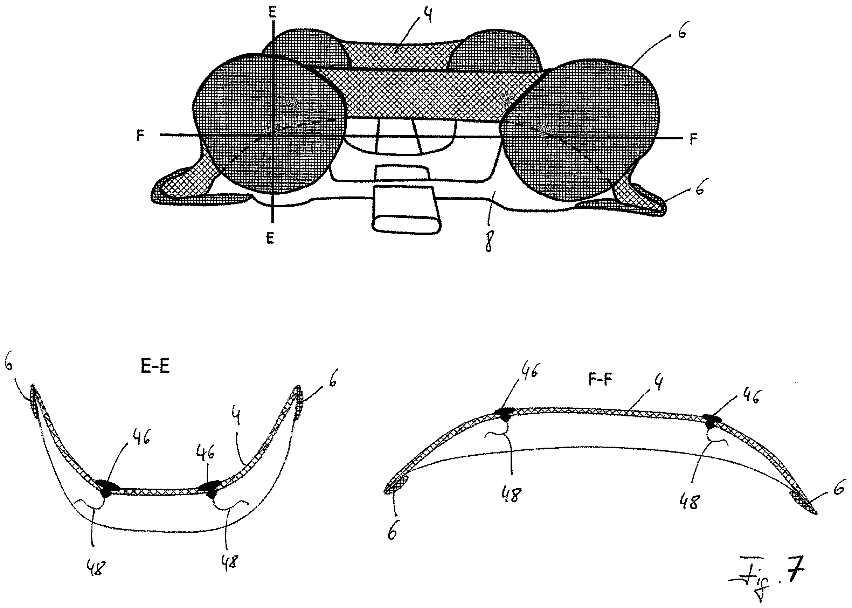

Die Figuren 6 und 7 zeigen die bereits aus Figur 1 bekannten Ansichten einer Einrichtung gemäß einem weiteren Ausführungsbeispiel der vorliegenden Erfindung, die sich von der in Figur 1 gezeigten Ausgestaltung unterscheidet. Das Auflageelement 4 ist an sechs Befestigungsstellen 6 an der Stützstruktur 8 befestigt. Insbesondere in den unteren beiden Schnittdarstellungen der Figur 6 ist ein Abspannelement 44 zu erkennen, das an einer weiteren Stelle das Auflageelement 4 mit der Stützstruktur 8 verbindet. Dadurch wird die Form des Auflageelementes 4 an dieser Stelle verändert und es kommt zu einer punktuellen Entlastung.

Figur 7 zeigt die gleichen Ansichten eines Teils einer weiteren Ausführung der vorliegenden Erfindung. In das Auflageelement 4 sind im gezeigten Ausführungsbeispiel Elektroden 46 eingesetzt, die im angelegten Zustand mit der Haut des Trägers in Kontakt kommen und so beispielsweise elektrische Signale von der Haut weg oder auf die Haut hin leiten können. Alternativ oder zusätzlich dazu können auch Sensoren verwendet werden, die mit der Haut in Kontakt kommen sollen. Über schematisch dargestellte elektrische Leitungen 48 werden die elektrischen Signale zur Elektrode 46 oder von der Elektrode 46 geleitet.

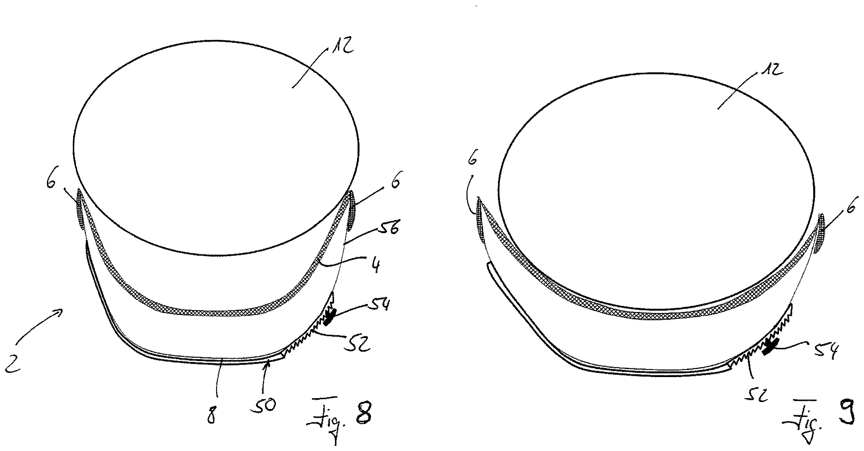

Die Figuren 8 und 9 zeigen eine weitenverstellbare Ausgestaltung der vorliegenden Erfindung. In Figur 8 ist ein Arm 12 dargestellt, der in ein Stützelement 2 gemäß einem Ausführungsbeispiel der vorliegenden Erfindung eingesetzt werden soll. Das Auflageelement 4 ist wie in den bereits gezeigten Ausführungsbeispielen über Befestigungsstellen 6 an der Stützstruktur 8 angeordnet. Diese Stützstruktur 8 ist im gezeigten Ausführungsbeispiel längenverstellbar. Ein erstes Bauteil 50 verfügt über eine Zahnreihe 52, in die ein Rastelement 54 eines zweiten Bauteils 56 eingreifen kann. Während in Figur 8 der Abstand zwischen den beiden dargestellten Befestigungsstellen 6 zu klein ist, um den Arm 12 aufzunehmen, wurde dies in Figur 9 geändert. Der Arm 12 passt zwischen die beiden Befestigungsstellen 6, da das Rastelement 54 in einen anderen Zahn der Zahnreihe 52 eingreift.

### Bezugszeichenliste:

- 2: Stützelement
- 4: Auflageelement
- 6: Befestigungsstelle
- 8: Stützstruktur
- 10: Vorsprung
- 12: Arm
- 14: Träger
- 16: Schiene
- 18: Gelenk
- 20: gestrichelte Linie
- 22: Riemen
- 24: Hebelelement
- 26: Gelenk
- 28: Kraftübertragungsvorrichtung
- 30: Gürtelelement
- 32: Kraftaufbringelement
- 36: Schalenelement
- 38: Betätigungselement
- 40: Endbereich
- 42: Gelenk
- 44: Abspannelement
- 46: Elektrode
- 48: elektrische Leitung
- 50: erstes Bauteil
- 52: Zahnreihe
- 54: Rastelement
- 56: zweites Bauteil

## Patentansprüche

1. Orthopädietechnische Einrichtung zum Stützen einer Extremität eines Trägers (14), wobei die Einrichtung wenigstens ein Stützelement (2) aufweist, auf dem in einem angelegten Zustand der Einrichtung die Extremität aufliegt, wobei das Stützelement (2) eine Stützstruktur (8) und ein Auflageelement (4) aufweist, wobei das Auflageelement (4) ein flächiges, flexibles, vorzugsweise elastisches Material aufweist oder daraus besteht, **dadurch gekennzeichnet, dass** das Auflageelement derart an der Stützstruktur (8) befestigt ist, dass es nicht vollflächig an der Stützstruktur anliegt, sondern sich zwischen dem Auflageelement und der Stützstruktur ein Spalt befindet.

2. Einrichtung nach Anspruch 1, **dadurch gekennzeichnet**, das das Auflageelement (4) an wenigstens drei, bevorzugt wenigstens vier, besonders bevorzugt wenigstens sechs Befestigungsstellen (6) an der Stützstruktur (8) befestigt ist oder an einer umlaufenden Befestigungsstelle (6), bevorzugt einem umlaufenden Rand der Stützstruktur (8) befestigt ist.

3. Einrichtung nach Anspruch 1 oder 2, **dadurch gekennzeichnet, dass** das Auflageelement (4) zumindest teilweise, vorzugsweise vollständig aus einem luftdurchlässigen Material hergestellt ist.

4. Einrichtung nach einem der vorstehenden Ansprüche, **dadurch gekennzeichnet, dass** das Auflageelement (4) zumindest teilweise, vorzugsweise vollständig aus einer Folie, einem Netz, einem Textil, beispielsweise einem luftdurchlässigen Gewirk, einem perforierten Polstermaterials, beispielsweise einem Schaumstoff oder einem Elastomer, oder einem auxetischen Material hergestellt ist.

5. Einrichtung nach einem der vorstehenden Ansprüche, **dadurch gekennzeichnet, dass** die Stützstruktur (8) ein Kunststoffbauteil, ein Metallbauteil oder ein Bauteil aus einem Faserverbundwerkstoff ist oder aufweist und die Befestigungsstellen (6) von einem Rest der Stützstruktur beispielsweise in Form von Laschen hervorstehen.

6. Einrichtung nach einem der vorstehenden Ansprüche, **dadurch gekennzeichnet, dass** das Auflageelement (4) lösbar an der Stützstruktur (8) befestigt ist.

7. Einrichtung nach einem der vorstehenden Ansprüche, **dadurch gekennzeichnet, dass** ein Abstand zwischen verschiedenen Befestigungsstellen (6), vorzugsweise durch ein Seilsystem oder Zugsystem, einstellbar ist

8. Einrichtung nach einem der vorstehenden Ansprüche, **dadurch gekennzeichnet, dass** die Einrichtung wenigstens einen, vorzugsweise angetriebenen, Aktuator aufweist, durch den ein Abstand zwischen wenigstens zwei verschiedenen Befestigungsstellen (6) veränderbar ist.

9. Einrichtung nach einem der vorstehenden Ansprüche, **dadurch gekennzeichnet, dass** die Einrichtung wenigstens ein zweites Stützelement (2) aufweist, das im angelegten Zustand der Einrichtung auf einer dem ersten Stützelement (2) abgewandten Seite der Extremität angeordnet ist.

10. Einrichtung nach einem der vorstehenden Ansprüche, **dadurch gekennzeichnet, dass** die Einrichtung eine Orthese, eine Lagerungsvorrichtung, ein Rehabilitationsgerät oder ein Exoskelett ist.

## Claims

1. Orthopedic device for supporting an extremity of a wearer (14), wherein the device comprises at least one support element (2), on which the extremity rests when the device is applied, wherein
the support element (2) features a support structure (8) and a contact element (4), wherein the contact element (4) comprises or is made of a flat, flexible, preferably elastic material, **characterized by the fact that** the contact element is fixed to the support structure (8) in such a way that it is not in full contact but creates a gap between the contact element and the support structure.

2. The device according to claim 1, **characterized by** the fact that the contact element (4) is fixed to the support structure (8) at at least three, preferably at least four, especially preferably at least six fixing points (6) or at a circumferential fixing point (6), preferably on a circumferential edge of the support structure (8).

3. The device according to claim 1 or 2, **characterized by** the fact that the contact element (4) is made at least partially, but preferably completely, from a material that is permeable to air.

4. The device according to one of the above claims, **characterized by** the fact that the contact element (4) is at least partially, but preferably completely, made of a foil, a mesh, a textile such as an air-permeable fabric, a perforated padding material such as a foam or an elastomer, or an auxetic material.

5. The device according to one of the above claims, **characterized by** the fact that the support structure (8) is or comprises a plastic component, a metal component or a component made of a fiber composite, and that the fixing points (6) protrude from the rest of the support structure, for example in the form of tabs.

6. The device according to one of the above claims, **characterized by** the fact that the contact element (4) is fixed to the support structure (8) such that it can be detached.

7. The device according to one of the above claims, **characterized by** the fact that a distance between different fixing points (6) can be adjusted, preferably by way of a cable system or a traction system.

8. The device according to one of the above claims, **characterized by** the fact that the device comprises at least one, preferably driven, actuator, by means of which a distance between the at least two different fixing points (6) can be changed.

9. The device according to one of the above claims, **characterized by** the fact that the device has at least a second support element (2), which is arranged on a side of the extremity that faces away from the first support element (2) when the device is applied.

10. The device according to one of the above claims, **characterized by** the fact that the device is an orthosis, a mounting device, a rehabilitation device or an exoskeleton.

## Revendications

1. Dispositif orthopédique pour soutenir un membre d'un porteur (14), le dispositif comprenant au moins un élément de soutien (2) sur lequel repose le membre, dans un état appliqué du dispositif, dans lequel l'élément de soutien (2) présente une structure de soutien (8) et un élément de support (4), l'élément de support (4) comprenant ou étant constitué d'un matériau plat, flexible, de préférence élastique,
**caractérisé en ce que** l'élément de support est fixé à la structure de soutien (8) de manière à ne pas s'appuyer par toute sa surface sur la structure de soutien, mais à former un intervalle entre l'élément de support et la structure de soutien.

2. Dispositif selon la revendication 1, **caractérisé en ce que** l'élément de support (4) est fixé à la structure de soutien (8) en au moins trois, de préférence au moins quatre, de manière particulièrement préférée au moins six emplacements de fixation (6) ou est fixé à un emplacement de fixation périphérique (6), de préférence à un bord périphérique de la structure de soutien (8).

3. Dispositif selon la revendication 1 ou 2, **caractérisé en ce que** l'élément de support (4) est réalisé au moins en partie, de préférence en totalité, d'un matériau perméable à l'air.

4. Dispositif selon l'une des revendications précédentes, **caractérisé en ce que** l'élément de support (4) est réalisé au moins en partie, de préférence en totalité, d'un film, d'un filet, d'un textile, par exemple d'un tricot perméable à l'air, d'un matériau de rembourrage perforé, par exemple d'une mousse ou d'un élastomère, ou d'un matériau auxétique.

5. Dispositif selon l'une des revendications précédentes, **caractérisé en ce que** la structure de soutien (8) est ou comprend un composant en matière plastique, un composant métallique ou un composant en matériau composite fibreux, et les emplacements de fixation (6) font saillie par rapport à un reste de la structure de soutien, par exemple sous forme de pattes.

6. Dispositif selon l'une des revendications précédentes, **caractérisé en ce que** l'élément de support (4) est fixé de manière amovible à la structure de soutien (8).

7. Dispositif selon l'une des revendications précédentes, **caractérisé en ce qu'**une distance entre différents emplacements de fixation (6) est réglable, de préférence par un système à câble ou par un système de traction.

8. Dispositif selon l'une des revendications précédentes, **caractérisé en ce que** le dispositif présente au moins un actionneur, de préférence entraîné, permettant de faire varier une distance entre au moins deux emplacements de fixation différents (6).

9. Dispositif selon l'une des revendications précédentes, **caractérisé en ce que** le dispositif comporte au moins un deuxième élément de soutien (2) qui, à l'état appliqué du dispositif, est disposé sur un côté du membre détourné du premier élément de soutien (2).

10. Dispositif selon l'une des revendications précédentes, **caractérisé en ce que** le dispositif est une orthèse, un dispositif de positionnement, un appareil de rééducation ou un exosquelette.
